# EUROPÄISCHE PATENTANMELDUNG

(11) **EP 0 750 846 A1**
(43) Veröffentlichungstag der Anmeldung: **02.01.1997**
(21) Anmeldenummer: 96110137.5
(22) Anmeldetag: 24.06.1996
(51) Int. Cl.: A23K 1/16

(54) **Verwendung von Balhimycin zur Leistungssteigerung bei Tieren sowie leistungssteigernde Mittel**

(30) Priorität: 28.06.1995 DE 19523394
(71) Anmelder: HOECHST AKTIENGESELLSCHAFT, 65929 Frankfurt am Main (DE)
(72) Erfinder: Lohner, Manfred Dr., 65343 Eltville (DE); Scheuermann, Stefan Dr., 65606 Villmar (DE); Vértesy, Laszlo Dr., 65817 Eppstein (DE)

(57) **Zusammenfassung**

Balhimycin, gegebenenfalls in Kombination mit einem oder mehreren seiner Derivate, eignet sich zur Leistungssteigerung bei mono- oder polygastrischen Tieren.

## Beschreibung

Die vorliegende Erfindung betrifft die Verwendung von Balhimycin zur Leistungssteigerung bei Tieren sowie leistungssteigernde Mittel.

Balhimycin, eine Verbindung der Formel I 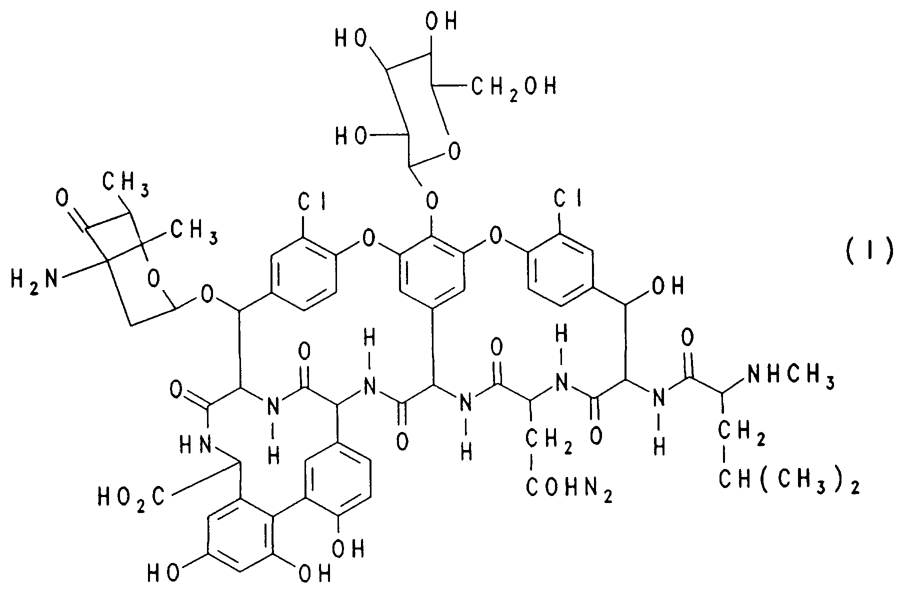 ist ein bekanntes Glycopeptidantibiotikum (vgl. EP 0 468 504). Derivate des Balhimycins und ihre Eignung als Leistungsförderer in der Landwirtschaft sind ebenfalls bekannt (vgl. EP 0 521 408). Die besonders stark ausgeprägte Eignung von Balhimycin zur Leistungssteigerung bei Tieren ist jedoch auch angesichts des Standes der Technik überraschend.

Zum Erfindungsgegenstand gehört demzufolge die Verwendung von Balhimycin gegebenenfalls in Kombination mit einem oder mehreren seiner Derivate zur Leistungssteigerung bei mono- oder polygastrischen Tieren, sowie Mittel zur Leistungssteigerung bei mono- oder polygastrischen Tieren, gekennzeichnet durch einen Gehalt an Balhimycin gegebenenfalls in Kombination mit einem oder mehreren seiner Derivate.

Balhimycin läßt sich herstellen durch Fermentation des Mikroorganismus Actinomycetes species DSM 5908 wie in der obengenannten EP 0 468 504 (US 5,451,570) beschrieben.

Die erfindungsgemäßen Mittel können wie erwähnt neben dem Balhimycin eines oder mehrere Balhimycin-Derivate enthalten; diese Balhimycin-Derivate sind insbesondere Des-Methyl-Balhimycin, Des-Methylleucyl-Balhimycin, Des-Gluco-Balhimycin, Ureido-Balhimycin, Des-Methyl-des-Gluco-Balhimycin, Methyl-Balhimycin, Balhimycin R oder Balhimycin V bzw. deren Hydrate und/oder physiologisch verträgliche Salze sein. Die Herstellung und Eigenschaften der genannten Balhimycin-Derivate sind in der EP 0 521 408 beschrieben, auf die an dieser Stelle ausdrücklich Bezug genommen wird.

Der Wirkstoff der erfindungsgemäßen Mittel besteht zu 50 bis 100 %, vorzugsweise 60 bis 100 %, insbesondere 70 bis 100 % aus Balhimycin.

Von besonderer Bedeutung sind die erfindungsgemäßen Mittel bei der Anwendung bei landwirtschaftlichen Nutztieren männlichen und weiblichen Geschlechts sowie kastrierten Tieren, wie Geflügel, Ferkeln, Mastschweinen, Zuchtschweinen, Kälbern, Mastrindern und Milchkühen. Die Mittel eignen sich auch als Leistungsförderer bei anderen Tieren, wie Kaninchen oder Fischen. Ganz besonders bevorzugt ist die Anwendung der erfindungsgemäßen Mittel bei Geflügel, Ferkeln und Mastschweinen, insbesondere bei Masthühnchen (Broilern) und Mastputen.

Als Leistungsförderung ist in diesem Zusammenhang insbesondere eine Verbesserung der Futterverwertung, eine Steigerung der Lebendgewichtszunahme, Erhöhung der Milchleistung oder Steigerung der Eierproduktion zu verstehen.

Der Wirkstoff wird den Tieren vorzugsweise in einer Dosierung von 0,1 bis 600 mg pro kg Körpermasse und Tag verabreicht. Der Wirkstoff kann auf vielfältige Art und Weise den Tieren verabreicht werden.

Die erfindungsgemäßen Mittel weisen eine unterschiedliche Zusammensetzung bzw. Darreichungsform auf.

Als erfindungsgemäß anstelle oder zusammen mit Balhimycin und ggf. dessen Derivaten kommen beispielsweise physiologisch verträgliche Salze und Ester des Balhimycins und seiner Derivate oder deren Kombination in Betracht.

Es ist beispielsweise möglich, den Wirkstoff als Reinsubstanz oder als Rohprodukt, beispielsweise nach der Extraktion der wirkstoffhaltigen Kulturbrühe oder in einer geeigneten Zubereitung dem Alleinfutter oder Ergänzungsfutter oder auch einem Teil der Tagesration zuzufügen. Das Futter kann dabei in flüssiger oder fester Form vorliegen. Alternativ kann man den Wirkstoff, die Wirkstoffkombination oder eine Wirkstoffzubereitung dem Trinkwasser zusetzen. Auch ist es möglich, den Wirkstoff oder die Wirkstoffe mit üblichen Hilfsmitteln in eine oral zu applizierende feste oder flüssige galenische Zubereitung zu bringen und als solche zu verabreichen oder dem Futter zuzusetzen. Auch eine parenterale Applikation des Wirkstoffs mit Hilfe eines Implantats ist möglich.

Eine besonders bevorzugte Form der Balhimycin und gegebenenfalls eines oder mehrere seiner Derivate enthaltenden Mittel läßt sich gewinnen, indem man Balhimycin durch Fermentation eines geeigneten Mikroorganismus (vgl. EP 0 468 504) herstellt und das Balhimycin zusammen mit nichtflüchtigen Bestandteilen der Kulturbrühe weiterverarbeitet. Die Gewinnung des Balhimycins zusammen mit den nichtflüchtigen Bestandteilen der Kulturbrühe erfolgt z.B. durch Sprühtrocknung der das Balhimycin enthaltenden Kulturbrühe. Auf diese Weise erhält man ein Produkt das abhängig vom Mikroorganismus, auch neben dem Balhimycin bestimmte Mengen an Balhimycin-Derivaten enthalten kann. Der Wirkstoffgehalt entsprechender Produkte kann weiterhin durch Hinzufügung von reinem Balhimycin bzw. einem oder mehreren seiner Derivate gezielt verändert werden.

Eine in vielen Fällen bevorzugte Applikationsform des Wirkstoffs ist die Zugabe zum Futter in Form eines Konzentrats (Praemix). Das Konzentrat kann beispielsweise durch Vermischen des Wirkstoffs oder der Wirkstoffkombination, des Rohprodukts oder des wirkstoffhaltigen Extrakts mit einem physiologisch verträglichen, festen oder flüssigen Träger hergestellt werden.

Als fester Trägerstoff kommen beispielsweise Getreidenebenprodukte, wie Weizennachmehl, Weizenkleie oder entölte Reiskleie, aber auch Maismehl, Sojamehl, Bolus alba oder Calciumcarbonat, in Betracht. Als flüssige Träger können physiologische Salzlösungen, Wasser und physiologisch verträgliche organische Lösungsmittel Verwendung finden. Dabei ist es möglich, geeignete Zusatzstoffe, wie Emulgatoren, Dispersions-, Suspensions-, Benetzungs- oder Geliermittel zu verwenden. Das Konzentrat (Praemix) enthält in der Regel 0,1 bis 20 Gew.-%, vorzugsweise 0,5 bis 15 Gew.-%, des Wirkstoffs oder der Kombination, wobei je nach Anwendungszweck die Wirkstoffkonzentration auch unter- oder überschritten werden kann.

Bei der Verabreichung mit Futtermitteln wird zweckmäßig so verfahren, daß ein Konzentrat mit dem Futter homogen vermengt wird. Bei den Futtermitteln eignen sich die üblicherweise verwendeten Futtermittel, wie beispielsweise verschiedene Getreide, Nachprodukte der Ölgewinnung (z.B. Sojaextraktionsschrot) und andere Energie- und Proteinträger, wie Tapioka und Fischmehl, sowie daraus hergestellte Futtermischungen, Ergänzungsfuttermittel oder Mineralstoffmischungen.

Die optimale Wachstumssteigerung hängt in der Regel von der Zusammensetzung des Futters, insbesondere dem Proteingehalt und von dem Gehalt an Balhimycin bzw. seinen Derivaten oder deren Kombination ab, wobei in der Regel bei steigendem Wirkstoffgehalt ein Maximum der Wachstumssteigerung durchlaufen wird. Die optimale Dosierung läßt sich durch eine faktorielle Versuchsplanung in relativ wenigen Vorversuchen leicht ermitteln. In der Regel ist die Konzentration des Wirkstoffes im Futter 0,1 bis 500 mg/kg, vorzugsweise 0,1 bis 200 mg/kg, insbesondere 0,1 bis 100 mg/kg.

Eine weitere Form der Verabreichung des Wirkstoffs besteht in der Lösung oder Suspension im Trinkwasser oder anderen Tränken, wie z.B. dem Milchaustauscher.

Die Verabreichung des Wirkstoffs kann auch in der Weise erfolgen, daß den Tieren, vorzugsweise während oder kurze Zeit vor oder nach der Fütterung, der Wirkstoff in Form seiner festen oder flüssigen galenischen Zubereitung, direkt oral verabreicht wird. Die galenische Zubereitung kann dabei z.B. eine Tablette, Kapsel, Paste, ein Granulat, Pulver, Bolus, Slow-Release Bolus, Saft, Sirup, Leckstein oder ein wie oben beschriebenes Praemix-Konzentrat sein. Insbesondere bei Weidetieren kann diese Applikationsform von besonderem Interesse sein.

Für die Verabreichung in Form von Tabletten, Kapseln, Pasten, Boli, Pillen, Granulaten, Säften, Sirups und ähnlichem können die gleichen Hilfs- und Zusatzstoffe verwendet werden, wie sie in der pharmazeutischen Technik bekannt sind, insbesondere Hilfsstoffe für Zubereitungen zur Langzeitapplikation mit kontinuierlicher Freisetzung des Wirkstoffs. Von besonderem Interesse sind Zubereitungen letzterer Art, die speziell für Wiederkäuer entwickelt worden sind. Der Wirkstoff kann beispielsweise mit pulverförmigen Verdünnungsmitteln, wie z.B.mikrokristalliner Cellulose, Zucker oder Stärke, zum Abfüllen des Kapselvolumens vermischt werden. Die Herstellung der Tabletten kann ebenfalls auf übliche Weise unter Zugabe von Substanzen, wie z.B. Cellulose, Lactose, Natriumchlorid, Stärke, Dextrin, Cellulosederivaten etc., erfolgen. Auch für die Herstellung von flüssigen Zubereitungen können die in der Pharmazie üblichen Hilfsstoffe, wie z.B.. Pflanzenöle, Kollidon, Cellulosederivate und ähnliches verwendet werden. Eine wäßrige Lösung oder Suspension von Balhimycin bzw. dessen Analoga kann/können z.B. neben dem Wirkstoff auch Nebenbestandteile aus dem Rohextrakt der Kulturbrühe sowie z.B. geeignete Puffersubstanzen enthalten. Pro Verabreichungseinheit soll vorzugsweise 0,1 bis 600 mg Wirkstoff bzw. Wirkstoffgemisch enthalten sein.

Bei der parenteralen Applikation des Wirkstoffs oder der Wirkstoffkombination werden die Wirkstoffe vorzugsweise mittels eines in üblicher Weise hergestellten und eingesetzten Implantats je nach Tierart über einige Wochen oder Monate kontinuierlich in der erforderlichen Dosis freigesetzt.

Auch Kombinationen mit anderen Zusatzstoffen, wie Antibiotika und Chemotherapeutika sind zur Verbesserung der Wirkung möglich.

Die erfindungsgemäß eingesetzten Wirkstoffe, vorzugsweise Balhimycin, beeinflussen die Leistung positiv. Darüber hinaus ist bei bedarfsgerechter Versorgung in der Regel eine geringere Menge an Futter für die gleiche Gewichtszunahme erforderlich wie sie ohne den Wirkstoff bzw. dessen Kombination benötigt wird.

Dadurch ist ein proteinsparender und futtersparender Effekt festzustellen. Damit werden in der Regel auch die Belastungen der Umwelt durch die Ausscheidungen der Tiere reduziert.

Durch die nachfolgenden Beispiele, Wirksamkeitsnachweise sowie durch den Inhalt der Patentansprüche soll die vorliegende Erfindung näher erläutert werden.

### Beispiel:

Ein Fütterungsversuch mit Balhimycin wurde mit 160 Mastküken (80 männl./80 weibliche) in Bodenhaltung mit 5 Gruppen durchgeführt:
- 1. Gruppe:: unbehandelte Kontrolle
- 2. Gruppe:: Balhimycin 15 mg/kg Futter
- 3. Gruppe:: Balhimycin 30 mg/kg Futter
- 4. Gruppe:: Balhimycin 45 mg/kg Futter
- 5. Gruppe:: Balhimycin 60 mg/kg Futter

Das Futter war wie folgt zusammengesetzt (Tabelle 1):

**Tabelle 1**

| Alleinfutter für Mastküken | |
|---|---|
| Zusammensetzung in % | |
| Fischmehl | 6,0 |
| Futterhefe | 2,0 |
| Sojaöl | 4,35 |
| Sojaschrot | 24,0 |
| Luzernegrünmehl | 1,0 |
| Mais | 44,615 |
| Weizen | 6,7 |
| Weizennachmehl | 8,5 |
| phosphors. Futterkalk | 1,4 |
| kohlens. Futterkalk | 0,96 |
| Spurenelementmischung für Geflügel | 0,04 |
| Viehsalz | 0,09 |
| Methionin DL | 0,07 |
| Raiffeisen Vitaminvorm. G 1 | 0,015 |
| Cholinchlorid | 0,26 |
| | 100 |

Die Tiere wurden in Buchten zu je 40 Tieren gehalten und erhielten die Futtermischungen der Gruppeneinteilung entsprechend 35 Tage. Die Gewishtsentwicklung, Futteraufnahme und Futterverwertung sind in Tabelle 2 dargestellt.

**Tabelle 2**

| Wachstum und Futterverwertung bei Broilern in Abhängigkeit von der Balhimycin-Konzentration des Futters | | | | | | |
|---|---|---|---|---|---|---|
| Gruppe | Anzahl n | Lebendmasse | | Zunahme g/Tag | Futter | |
| | | Anfang g | Ende g | | aufnahme g/Tag | verwertung kgFA/kgLM* |
| A Kontrolle | | 41,3 | 1482,0 | 41,17 *c* | 72,6 | 1,76 |
| | 152,00 | | 139,8 | 3,95 | 4,12 | 0,03 |
| | | 100,0 | 100,0 | 100,0 | 100,0 | 100,0 |
| B Balhimycin 15 mg/kg | | 40,0 | 1602,0 | 44,63 *b* | 72,5 | 1,62*** |
| | 151,00 | | 130,5 | 3,73 | 0,78 | 0,02 |
| | | 96,9 | 108,1 | 108,4 | 99,9 | 92,0 |
| C Balhimycin 30 mg/kg | | 43,5 | 1640,0 | 45,62 *a* | 75,3 | 1,65** |
| | 156,00 | | 125,4 | 3,59 | 1,33 | 0,04 |
| | | 105,3 | 110,7 | 110,8 | 100,6 | 93,8 |
| D Balhimycin 45 mg/kg | | 42,0 | 1617,0 | 44,99 *ab* | 73,0 | 1,62*** |
| | 154,00 | | 134,7 | 3,85 | 1,73 | 0,02 |
| | | 101,7 | 109,1 | 109,3 | 100,6 | 92,0 |
| E Balhimycin 60 mg/kg | | 42,3 | 1600,0 | 44,50 *b* | 73,1 | 1,64** |
| | 156,00 | | 141,1 | 4,02 | 2,29 | 0,03 |
| | | 102,4 | 108,0 | 108,1 | 100,7 | 93,2 |
| unterschiedliche Buchstaben bedeuten signifikante unterschiede zwischen den Gruppen (P < 0,05) | | | | | | |

| | | | | | | |
|---|---|---|---|---|---|---|
| Futterverwertung: ***P < 0.001 | | | | | | |
| **p < 0.005 signifikant gegenüber der Kontrolle | | | | | | |
| * kg FA/kg LM = kg Futteraufnahme / kg Lebendmassezunahme | | | | | | |

## Patentansprüche

1. Verwendung von Balhimycin, gegebenenfalls in Kombination mit einem oder mehreren seiner Derivate, zur Leistungssteigerung bei mono- oder polygastrischen Tieren.

2. Mittel zur Leistungssteigerung bei mono- oder polygastrischen Tieren, gekennzeichnet durch einen Gehalt an Balhimycin, gegebenenfalls in Kombination mit einem oder mehreren seiner Derivate.

3. Mittel gemäß Anspruch 2 zur Verabreichung in einer Dosis von 0,1 bis 600 mg Wirkstoff pro kg Körpermasse und Tag.

4. Mittel gemäß einem oder mehreren der Ansprüche 2 bis 3, dadurch gekennzeichnet, daß es mit festem oder flüssigem Allein- oder Ergänzungsfutter oder mit dem Trinkwasser zu verabreichen ist.

5. Mittel gemäß einem oder mehreren der Ansprüche 2 bis 4, dadurch gekennzeichnet, daß es ein festes oder flüssiges Konzentrat (Praemix) darstellt.

6. Mittel gemäß einem oder mehreren der Ansprüche 2 bis 4, dadurch gekennzeichnet, daß es eine oral zu applizierende, feste oder flüssige galenische Zubereitung, vorzugsweise eine Tablette, Kapsel, Paste, ein Granulat, ein Pulver, einen Saft, einen Sirup, einen Leckstein, einen Bolus oder Slow-Release-Bolus, darstellt.

7. Mittel gemäß Anspruch 2, dadurch gekennzeichnet, daß es ein Implantat zur parenteralen Applikation von Balhimycin und gegebenenfalls eines oder mehrerer seiner Derivate mit einer kontinuierlichen Freisetzung über einige Wochen oder Monate darstellt.

8. Mittel gemäß einem oder mehreren der Ansprüche 2-4, dadurch gekennzeichnet, daß es Balhimycin zusammen mit den nichtflüchtigen Bestandteilen der Balhimycin-Fermentation enthält.

9. Verfahren zur Herstellung eines Mittels gemäß einem oder mehreren der Ansprüche 2-8, dadurch gekennzeichnet, daß Balhimycin, gegebenenfalls in Kombination mit einem oder mehreren seiner Derivate, gegebenenfalls mit physiologisch verträglichen Hilfsstoffen in eine geeignete Darreichungsform gebracht wird.
